(19) Europäisches Patentamt
European Patent Office
Office européen des brevets

(11) Veröffentlichungsnummer **0 053 307**
**B1**

(12) # EUROPÄISCHE PATENTSCHRIFT

(45) Veröffentlichungstag der Patentschrift: **27.06.84**

(51) Int. Cl.³: **C 07 D 233/60**, C 07 D 249/08, A 01 N 43/64, A 01 N 43/50

(21) Anmeldenummer: **81109619.7**

(22) Anmeldetag: **11.11.81**

(54) **Pestizide Imidazol- und Triazolderivate.**

(30) Priorität: **29.11.80 DE 3045055**

(43) Veröffentlichungstag der Anmeldung: **09.06.82 Patentblatt 82/23**

(45) Bekanntmachung des Hinweises auf die Patenterteilung: **27.06.84 Patentblatt 84/26**

(84) Benannte Vertragsstaaten: **AT BE CH DE FR GB IT LI LU NL**

(56) Entgegenhaltungen:
**FR - A - 2 103 452**
**FR - A - 2 209 581**
**FR - A - 2 355 835**
**GB - A - 2 010 270**
**US - A - 3 989 711**

(73) Patentinhaber: **CELAMERCK GmbH & Co. KG, Binger Strasse 173, D-6507 Ingelheim am Rhein (DE)**

(72) Erfinder: **Curtze, Jürgen, Dr., Rheingaublick 6, D-6222 Geisenheim-Johannisberg (DE)**
Erfinder: **Mengel, Rudolf, Dr., Schützenpfad 22, D-6507 Ingelheim am Rhein (DE)**
Erfinder: **Becher, Heinz-Manfred, Dr., Pfarrer-Heberer-Strasse 5, D-6530 Bingen am Rhein (DE)**
Erfinder: **Drandarevski, Christo Assenov, Dr., Velt-Stoss-Strasse 17, D-6507 Ingelheim am Rhein (DE)**
Erfinder: **Lust, Sigmund, Dr., Klappacher Strasse 2f, D-6100 Darmstadt (DE)**

**EP 0 053 307 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue heterocyclische Verbindungen mit bioziden Eigenschaften.
Aus der FR-A- 2 355 835 sind fungizide und nematizide Wirkstoffe der Formel

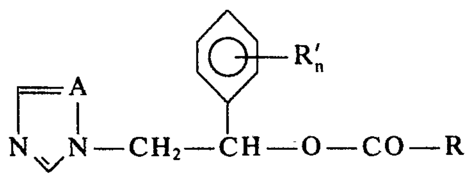

bekannt, worin A CH oder N, n eine ganze Zahl von 0 bis 5, R Alkyl, Alkenyl, Alkinyl, Cycloalkyl, Halogenalkyl, ggf. substituiertes Phenyl, Phenylalkyl oder Phenoxyalkyl, Amino, Alkylamino, Dialkylamino, Alkyl-alkylcarbonylamino oder ggf. substituiertes Phenylamino und R' Halogen, Alkyl, Alkoxy, Alkylthio, Alkylsulfonyl, Halogenalkyl, Nitro, Cyano, ggf. substituiertes Phenyl oder ggf. substituiertes Phenoxy bedeuten. Diese Verbindungen leiten sich von einem Aminoalkohol ab, der die Grundstruktur

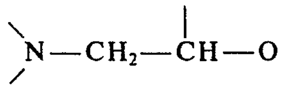

enthält.

Wie nun gefunden wurde, zeigen die neuen Verbindungen der nachstehenden Formel I eine sehr gute biozide, insbesondere fungizide Wirkung. Bei den neuen Verbindungen handelt es sich um Aldehydderivate (Aminale), deren Wirkung wegen des wesentlichen Strukturunterschiedes nicht vorherzusehen war.

Die neuen Verbindungen entsprechen der allgemeinen Formel

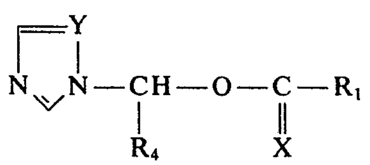

(I)

worin

$R_1$ Phenyl; Naphthyl; einen durch Halogen, Phenyl, Nitro, Cyano, $C_1$–$C_4$-Alkyl, $C_1$–$C_4$-Alkoxy, Phenoxy, $N(C_1-C_4\text{-Alkyl})_2$, $C_1$–$C_4$-Alkylthio, ein- bis dreifach ggf. gemischt substituierten Phenylrest; einen ggf. chlorsubstituierten $C_1$–$C_{10}$-Alkylrest; einen ggf. durch $C_1$–$C_4$-Alkyl oder Phenyl substituierten $C_3$–$C_7$-Cycloalkylrest; oder eine $NR_2R_3$-Gruppe;

$R_2$ Wasserstoff oder $C_1$–$C_4$-Alkyl;

$R_3$ $C_1$–$C_4$-Alkyl, Phenyl oder einen ein- bis dreifach durch Halogen, Nitro, Trifluormethyl, $C_1$–$C_4$-Alkyl- oder -Alkoxy ggf. gemischt substituierten Phenylrest;

$R_4$ Phenyl; einen durch 1 bis 2 Halogenatome, die Phenyl- oder Nitrogruppe substituierten Phenylrest; einen $C_1$–$C_4$-Alkylrest; einen Alkenylrest mit bis zu 10 C-Atomen; einen durch einen oder zwei Phenylreste (die ggf. durch Halogen- oder Nitrogruppen substituiert sein können) substituierten $C_1$–$C_{10}$-Alkylrest; oder einen ggf. durch $C_1$–$C_4$-Alkyl, $C_2$–$C_4$-Alkenyl, Benzyl oder Phenyl substituierten $C_3$–$C_7$-Cycloalkylrest;

X O oder S;
und

Y CH oder N

bedeutet. Sie können als Racemate, in Form der einzelnen Enantiomeren oder ggf. von diastereomeren Antipodenpaaren, als freie Verbindungen oder als Kupferkomplexe (Azol/Kupfer im Molverhältnis 2 : 1) vorliegen.

Im Rahmen der obigen Definitionen bedeutet $R_1$ bevorzugt Phenyl oder substituiertes Phenyl oder $NR_2R_3$, $R_4$ Alkyl, Y CH und N. Soweit in den Substituenten Kohlenstoffketten enthalten sind, können diese unverzweigt oder verzweigt sein. Typische Bedeutungen von $R_4$, soweit es einen substituierten Phenylrest bedeutet, sind 4-Nitrophenyl, 2-Chlorphenyl, 2,4-Dichlorphenyl, 2,6-Dichlorphenyl, 2-Bromphenyl. Unter »Halogen« sind Fluor, Chlor, Brom und Jod zu verstehen. Als Substituenten der Phenylgruppe sind im Fall $R_4$ gleich substituiertes Phenyl von den Halogenen Chlor und Brom bevorzugt. Bedeutet $R_1$ eine $NR_2R_3$-Gruppe, so können die Reste $R_2$ und $R_3$ gleich oder verschieden sein.

Die Verbindungen der Formel I werden nach neuartigen Reaktionen erhalten.

1.) Man setzt ein Acyl- oder Thioacyloxyhalogenmethan der Formel

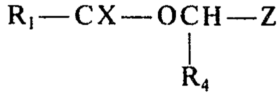

(II)

worin $R_1$, $R_4$ und X die obige Bedeutung haben und Z für Chlor oder Brom steht, mit einem Imidazol- oder Triazolsalz der Formel

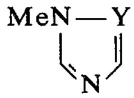

(III)

worin Me für ein Äquivalent eines Metallkations steht, in einem trockenen organischen Lösungsmittel um.

Als Lösungsmittel können beispielsweise Aceton, Acetonitril, Tetrahydrofuran oder Toluol dienen; bevorzugt wird Acetonitril verwendet. Die Reaktionstemperatur kann zwischen etwa 0°C und etwa 120°C liegen. Da die Reaktionsgeschwindigkeit stark von der Temperatur abhängt, ist es zweckmäßig, während der Umsetzung zu erwärmen. Nach dem Zusammengeben der Reaktionspartner scheidet sich das entstandene anorganische Salz ab. Es wird nach der Umsetzung abgetrennt, das Lösungsmittel abgedampft. Das zurückbleibende Reaktionsprodukt der Formel I wird gewünschtenfalls anschließend z. B. durch Umkristallisieren, Destillieren oder Säulenchromatographie gereinigt. In der organischen Lösung des Reaktionsprodukts vorhandener Aldehyd kann durch Ausschütteln mit wässeriger Natriumbisulfitlösung, saure organische Bestandteile können durch Ausschütteln mit Natriumbicarbonat entfernt werden, bevor man das organische Lösungsmittel abdampft.

2.) Man setzt eine Verbindung der Formel

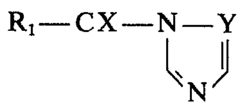

(IV)

(worin $R_1$, X und Y die obige Bedeutung haben, wobei X bevorzugt O ist) in einem trockenen organischen Lösungsmittel mit einem Aldehyd der Formel

$R_4$—CHO (V)

um.

Als Lösungsmittel können z. B. Toluol, Benzol, Aceton, Acetonitril, Tetrahydrofuran verwendet werden; bevorzugt wird Toluol. Die Reaktionstemperatur liegt zwischen der Umgebungstemperatur und der Siedetemperatur des Reaktionsgemisches, im allgemeinen zwischen 20°C und 150°C. Zweckmäßig erfolgt die Umsetzung bei der Siedetemperatur. Das nach dem Abdestillieren des Lösungsmittels erhaltene Reaktionsprodukt wird gewünschtenfalls wie bei Verfahren 1.) beschrieben gereinigt.

Statt einzelner Lösungsmittel können auch Lösungsmittelgemische verwendet werden.

Racemate können anschließend gewünschtenfalls in die optischen Antipoden aufgetrennt werden. Sofern die erhaltenen Verbindungen zwei asymmetrische C-Atome aufweisen, können sie aufgrund ihrer unterschiedlichen Löslichkeit in die diastereomeren Antipodenpaare aufgetrennt werden.

Gewünschtenfalls werden die Verbindungen der Formel I durch Umsetzung mit Kupfersalzlösungen in Kupferkomplexe (Molverhältnis Azol/Kupfer gleich 2 : 1) übergeführt.

Die Ausgangsstoffe werden nach üblichen Verfahren erhalten.

Die neuen Verbindungen eignen sich aufgrund ihrer bioziden Eigenschaften für die Verwendung bei der Bekämpfung von pflanzenschädigenden Organismen, insbesondere von phytopathogenen Pilzen. Hervorzuheben sind Mehltauarten und Rostpilze. Für diese Anwendung kommen außer den freien Wirkstoffen auch Komplexverbindungen der neuen Verbindungen mit Kupferionen in Betracht.

Für die Anwendung im Pflanzenschutz werden die neuen Verbindungen bzw. ihre Komplexverbindungen in üblicher Weise mit Hilfs- und/oder Trägerstoffen zu gebräuchlichen Formen von Schädlingsbekämpfungsmitteln verarbeitet, z. B. zu Lösungen, Lösungs- bzw. Emulsionskonzentraten, Suspensionspulvern, Stäuben, Emulsionen. Die Konzentrate werden vor der Anwendung ggf. mit Wasser verdünnt, so daß Spritzbrühen mit einem Wirkstoffgehalt zwischen etwa 0,005 und 1 Gewichtsprozent erhalten werden. Bei der Anwendung als Low-Volume- oder Ultra-Low-Volume-Formulierung kann der Wirkstoffgehalt auch erheblich höher sein (bei 20 bzw. 50 Gewichtsprozent).

Beispiele für erfindungsgemäße Formulierungen:

1. Suspensionspulver

| | |
|---|---|
| 20 Gew.-Teile | 1-[1-(3,4-Dichlorbenzoyloxy)-2,2-dimethyl-1-pentyl]-1,2,4-triazol |
| 20 Gew.-Teile | Kaolin |
| 5 Gew.-Teile | Natriumsulfat |
| 2 Gew.-Teile | Schlämmkreide |
| 9 Gew.-Teile | Calciumligninsulfonat (Dispergiermittel) |
| 1 Gew.-Teile | Diisobutylnaphthalin-natriumsulfonat (Netzmittel) |
| 43 Gew.-Teile | Kieselkreide |

Die Bestandteile werden vermahlen und das Mittel wird für die Anwendung in so viel Wasser suspendiert, daß die Wirkstoffkonzentration etwa 0,005 bis 0,5 Gewichtsprozent beträgt.

2. Emulsionskonzentrat

| | |
|---|---|
| 15 Gew.-Teile | 1-[1-(4-Chlorbenzoyloxy)-2,2-dimethyl-1-pentyl]-imidazol |
| 10 Gew.-Teile | Dodecylbenzolsulfonsäure-triäthylaminsalz |
| 75 Gew.-Teile | Dimethylformamid |

Die starke Wirkung der erfindungsgemäßen Verbindungen geht aus den folgenden Versuchsergebnissen hervor. Untersucht wurde die Wirkung gegen Mehltau an Gurken. Die Bewertung erfolgte mit den Noten 1–3.

Es bedeutet:

1 : Befall 0– 10%
2 : Befall 11– 40%
3 : Befall 41–100%

| Beispiel | Anwendungskonzentration | Bewertungsnote |
|---|---|---|
| 9 | 2 ppm | 1 |
| 15 | 2 ppm | 1 |
| 17 | 2 ppm | 2 |
| 33 | 2 ppm | 1 |
| 34 | 10 ppm | 1 |

Die folgenden Beispiele sollen die Herstellung der erfindungsgemäßen Verbindungen erläutern.

A. Herstellung von Ausgangsstoffen

(a) 1,2,4-Triazol-natrium

Die Lösung von 11,5 g Natrium in 300 ml Methanol wird 1/2 Stunde am Rückfluß gekocht, danach im Wasserstrahlvakuum zur Trockene eingedampft. Anschließend wird eine Mischung aus 34,5 g 1,2,4-Triazol und 100 ml Toluol zugegeben und im Wasserstrahlvakuum bis zur Gewichtskonstanz auf einem Wasserbad von ca. 80°C eingedampft. Der feste Rückstand, 1,2,4-Triazol-natrium, wird für die weitere Verwendung fein gemahlen. Ausbeute quantitativ.

(b) Imidazol-kalium

Aus 19,5 g Kalium in 500 ml Isopropyl-alkohol wird das Alkoholat hergestellt und entsprechend (a) mit 34 g Imidazol in 100 ml Toluol umgesetzt. Ausbeute quantitativ.

(c) 1-(3,4-Dichlorbenzoyloxy)-1-chlor-2,2-dimethylpentan

Zu 21,0 g 3,4-Dichlorbenzoylchlorid, gelöst in 70 ml Wasser- und alkoholfreiem Chloroform, werden 10 Tropfen zinkchloridgesättigtes Aceton gegeben. Unter Rühren tropft man innerhalb einer Minute 11,4 g 2,2-Dimethylpentanal zu. Die Mischung wird zunächst ohne Wärmezufuhr 15 Minuten, dann bei 40—42°C Innentemperatur 2 1/2 Stunden gerührt. Nach dem Abkühlen auf Raumtemperatur wird abgesaugt, das Filtrat über eine mit basischem Aluminiumoxid beschickte Säule gegeben und mit 20 ml Chloroform nachgewaschen. Die Lösung wird im Wasserstrahlvakuum auf dem Wasserbad bei 30°C Badtemperatur bis zur Gewichtskonstanz eingedampft. Die erhaltene Verbindung ist ein hellgelbes Öl; Ausbeute 92% d. Th.

(d) 1-Chlor-1-pivaloyloxy-2,2-dimethyl-3-phenylpropan

Eine Lösung von 12,1 g Pivalinsäurechlorid, mit $ZnCl_2$ gesättigt, in 50 ml Chloroform (wasser- und alkoholfrei) wird auf −10°C abgekühlt. Unter Rühren gibt man eine Lösung von 16,2 g 2,2-Dimethyl-3-phenylpropion-aldehyd in 10 ml Chloroform (wasser- und alkoholfrei) derart zu, daß die Temperatur von −10°C gehalten werden kann. Die Mischung wird dann 2 1/2 Stunden bei −5 bis −10°C gerührt. Danach wird das Gemisch über eine mit basischem Aluminiumoxid gefüllte, auf −10 bis −15°C gekühlte Säule gegeben. Man wäscht mit 20 ml Chloroform nach und dampft das Filtrat im Wasserstrahlvakuum bei 20°C Wasserbadtemperatur bis zur Gewichtskonstanz ein.

Ausbeute 25,8 g farbloses Öl (91,3% d. Th.).

(e) 1-(1-Methylcylohexylcarbonyl)-imidazol

Zu einer Lösung von 5,0 g 1-Methyl-1-cyclohexan-carbonsäure in 50 ml absolutem Toluol werden 5,7 g N,N'-Carbonyldiimidazol portionsweise zugegeben. Man läßt die Mischung 3 Stunden stehen und schüttelt zwischendurch öfters um. Die erhaltene Lösung der Titelverbindung wird als solche für die weitere Umsetzung benutzt.

Entsprechend (a) bis (e) können auch die übrigen Ausgangsstoffe gewonnen werden.

B. Herstellung von Verbindungen der Formel I

Beispiel 1

1-[1-(3,4-Dichlorbenzoyloxy)-2-2-dimethyl-1-pentyl]-1,2,4-triazol

Zu einer Lösung von 14,8 g 1-(3,4-Dichlorbenzoyloxy)-1-chlor-2,2-dimethylpentan in 70 ml Acetonitril abs. werden unter kräftigem Rühren und Kühlen mit Wasser 4,2 g 1,2,4-Triazol-natrium auf einmal zugegeben. Man rührt zunächst 15 Minuten bei Raumtemperatur, dann 1 Stunde bei 80°C Innentemperatur und schließlich noch 2 Stunden ohne Wärmezufuhr. Nach dem Abkühlen wird filtriert und aus dem Filtrat im Wasserstrahlvakuum bei 80°C Badtemperatur das Lösungsmittel entfernt. Der Rückstand wird in 30 ml Methanol gelöst und bis zur starken Trübung mit Wasser versetzt. Man rührt dann 1 Stunde im Eisbad, saugt das Kristallisat ab und wäscht es mit Wasser/Methanol 3 : 7.

Ausbeute 9,5 g (58,3% d. Th.); Fp. 102°C.

Analyse:

| | | | |
|---|---|---|---|
| ber.: | C 53,93% | H 5,34% | N 11,80% |
| gef.: | C 54,23% | H 5,41% | N 11,50% |

Bei der Umsetzung anderer Komponenten entsprechend diesem Beispiel werden z.T. Öle erhalten, die über eine mit Kieselgel gefüllte Säule in bekannter Weise gereinigt werden können; Laufmittel z. B. Toluol/Aceton 7 : 3. Die erhaltenen sauberen Fraktionen werden auf dem Wasserbad bei ca. 80°C im Wasserstrahlvakuum bis zur Gewichtskonstanz eingedampft.

Beispiel 2

1-[1-(3,4-Dichlorbenzoyloxy)-2,2-dimethyl-1-pentyl]-imidazol

Man setzt entsprechend Beispiel 1 14,8 g 1-(3,4-Dichlorbenzoyloxy)-1-chlor-2-2-dimethylpentan und 4,9 g Imidazol-kalium in 70 ml absolutem Acetonitril um. Das Reaktionsprodukt wird über eine mit

Kieselgel gefüllte Säule gereinigt. Man erhält 8,7 (54% d. Th.) eines dickflüssigen gelben Öls. Rf-Wert: 0,70 (bestimmt wie weiter unten angegeben).

Analyse:
ber.: C 57,46% H 5,63% N 7,89%
gef.: C 57,64% H 5,79% N 7,52%

Beispiel 3

1-[1-(Pivaloyloxy)-2,2-dimethyl-3-phenyl-1-propyl]-1,2,4-triazol

12,9 g 1-Chlor-1-pivaloyloxy-2,2-dimethyl-3-phenylpropan und 4,2 g 1,2,4-Triazol-natrium werden entsprechend Beispiel 1 in 70 ml absolutem Acetonitril umgesetzt. Die Reinigung erfolgt wie bei Beispiel 2 über eine Kieselgel-Säule. Man erhält ein fast farbloses Öl, das mit der Zeit kristallisiert; Fp. 67–68°C. Ausbeute: 7,4 g (51,3% d. Th.).

Für die Herstellung von Verbindungen mit R gleich Dialkylaminogruppe läßt sich Dialkylcarbamoylchlorid entsprechend einem aliphatischen Säurechlorid einsetzen.

Beispiel 4

1-[1-(Pivaloyloxy)-2,2-dimethyl-3-phenyl-1-propyl]-imidazol

12,9 1-Chlor-1-pivaloyloxy-2,2-dimethyl-3-phenylpropan und 4,9 g Imidazol-kalium werden in 70 ml absolutem Acetonitril entsprechend Beispiel 1 umgesetzt und über eine Kieselgel-Säule gereinigt. Man erhält 8,1 g eines farblosen Öls (56,6% d. Th.).

Das Öl kristallisiert sich nach einigem Stehen; Fp. 80–82°C.

Beispiel 5

1-[4-Nitro-α-(1-methylcyclohexylcarbonyloxy)-benzyl]-imidazol

Zu der gemäß A. (e) erhaltenen Lösung von 1-(1-Methylcyclohexylcarbonyl)-imidazol werden 5,3 g 4-Nitrobenzaldehyd auf einmal zugegeben. Die Mischung wird 6 Stunden unter Rückfluß gekocht. Nach Abkühlung auf Raumtemperatur wird mit 30 ml gesättigter wässeriger $NaHSO_3$-Lösung 1/2 Stunde kräftig gerührt, abgesaugt, der Rückstand mit Toluol gewaschen und die organische Phase abgetrennt. Sie wird mit Natriumhydrogencarbonatlösung, dann Wasser ausgeschüttelt und mit Magnesiumsulfat getrocknet. Man reinigt die Lösung über eine mit Kieselgel gefüllte Säule und dampft auf einem Wasserbad bei 80°C im Wasserstrahlvakuum bis zur Gewichtskonstanz ein. Man erhält 10,8 g eines braunen zähflüssigen Öls (89,5% d. Th.) Rf-Wert: 0,71 (Bestimmung siehe unten).

Beispiel 6

Kupferkomplex von 1-(2,4-Dichlor-α-pivaloyloxybenzyl)imidazol

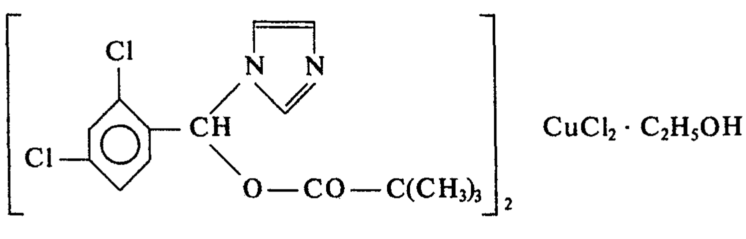

Die Lösungen von 5,0 g 1-(2,4-Dichlor-α-pivaloyloxybenzyl)-imidazol in 10 ml Ethanol und von 1,3 g $CuCl_2 \cdot 2H_2O$ in 15 ml Ethanol werden zusammengegeben. Eine Probe der Mischung wird nach Zugabe von Benzin (Siedebereich 40–60°C) bis zur deutlichen Trübung versetzt und das Reaktionsprodukt zur Kristallisation gebracht. Die Hauptmenge wird nun ebenfalls mit Benzin versetzt bis zur Trübung und angeimpft. Unter Rühren wird weiter Benzin zugesetzt, bis die Kristallabscheidung vollständig ist. Man rührt noch 1 Stunde bei Raumtemperatur, saugt ab, wäscht Benzin, dann, nach dem Trocknen, mit Ethanol/Wasser.

Ausbeute 4,6 g (72% der Theorie); Fp.: 187°C.

## Beispiel 7

### Dimethylthiocarbaminsäure-O-(α,2,4-trichlorbenzylester) (I)

9,89 g (80 mMol) Dimethylthiocarbamoylchlorid werden in 80 ml Chloroform gelöst, als Katalysator 10 Tropfen $ZnCl_2$-gesättigtes Aceton zugesetzt und auf −5°C abgekühlt. Unter Rühren werden innerhalb von 15 Minuten 14,0 g (80 mMol) 2,4-Dichlorbenzaldehyd eingetragen. Es wird 1/2 Stunde bei −5°C, dann 1 1/2 Stunde bei Raumtemperatur nachgerührt. Zur Entfernung des Katalysators wird die Lösung auf −10°C abgekühlt und in einer Säule über 20 g bas. $Al_2O_3$ filtriert, mit 50 ml kaltem Chloroform nachgewaschen. Das Filtrat wird bei 25—30°C am Rotationsverdampfer im Vakuum eingedampft.
Ausbeute: 23,5 g langsam erstarrendes Öl.

### 1-(2,4-Dichlor-α-dimethylthiocarbamoyloxybenzyl)-1,2,4-triazol (II)

3,65 g (40 mMol) 1,2,4-Triazol-Natrium werden mit 30 ml Acetonitril gerührt, auf −5°C abgekühlt und innerhalb 15 Minuten eine Lösung von 11,95 g (40 mMol) I in 20 ml Acetonitril zugetropft. Es wird 30 Minuten bei −5°C, dann 1 Stunde bei Raumtemperatur nachgerührt, anschließend kurzzeitig auf 60°C erwärmt. Nach dem Stehen über Nacht wird im Vakuum das Acetonitril abdestilliert, der ölige Rückstand mit je 75 ml Toluol und Wasser geschüttelt. Die Toluollösung wird 30 Minuten mit gesättigter $NaHSO_3$-Lösung, dann mit verdünnter $Na_2CO_3$-Lösung gerührt, anschließend mit Wasser verdünnter $Na_2CO_3$-Lösung gerührt, anschließend mit Wasser ausgeschüttelt. Die Toluollösung wird mit $Na_2SO_4$ getrocknet, das Toluol im Vakuum abdestilliert, dabei 8,2 g dunkelbraunes Öl erhalten. Dieses Öl wird in 10 ml Toluol gelöst und über eine Kieselgel-Säule gereinigt. Eluiert wird mit Toluol-Aceton (70 : 30). Die Fraktionen mit der Substanz vom Rf-Wert 0,52 (DC in Toluol — Aceton 70 : 30) werden gesammelt und im Vakuum eingedampft.
Ausbeute: 3,6 g Öl, erstarrend, Fp. 129—130°C.

## Beispiel 8

### 1-(4-Nitro-α-phenylcarbamoyloxybenzyl)-imidazol

3,74 g (20 mMol) Imidazol-N-carbonsäureanilid und 3,02 g (20 mMol) 4-Nitrobenzaldehyd werden mit 25 ml abs. Toluol 6 Stunden unter Rückfluß gekocht. Nach dem Stehen über Nacht bei Raumtemperatur wird die überstehende Mutterlauge abdekantiert, das mit dunkelbrauner Schmiere durchsetzte Kristallisat mit Methanol verrührt. Die dabei erhaltenen Kristalle werden aus Dimethylformamid-Methanol umkristallisiert.
Ausbeute: 3,4 g; Fp. 167°C .

Entsprechend den obigen Beispielen werden auch die Verbindungen der nachstehenden Tabelle erhalten. Die angegebenen Rf-Werte werden im Dünnschichtchromatogramm (DC) bestimmt. Verwendet wurden DC-Fertigplatten der Fa. E. MERCK, Darmstadt, mit Kieselgel 60-F-254. Die Flecken sind im ultravioletten Licht bei 254 μm sichtbar oder durch Besprühen mit Dragendorffs Reagenz sichtbar zu machen. Als Laufmittel wurde Toluol/Ethanol 70 : 30 verwendet, Temperatur 20°C. Verbindungen mit mehr als einem optisch aktiven Zentrum zeigen häufig einen Doppelfleck, da sie aus zwei diastereomeren Antipodenpaaren bestehen.

Tabelle 1

(Verbindungen der Formel I, X gleich Sauerstoff)

| Beispiel | $R_1$ | $R_4$ | Y | Fp. | Rf-Wert |
|---|---|---|---|---|---|
| 7 | $4\text{-Cl}-C_6H_4$ | $(CH_3)_3C$ | N | 126° | |
| 8 | desgl. | desgl. | CH | 134° | |
| 9 | desgl. | $CH_3CH_2CH_2(CH_3)_2C$ | CH | 96° | |
| 10 | desgl. | desgl. | N | Öl | 0,74 |
| 11 | $4\text{-F}-C_6H_4$ | desgl. | N | Öl | 0,73 |
| 12 | desgl. | desgl. | CH | Öl | 0,66 |
| 13 | $4\text{-Br}-C_6H_4$ | desgl. | N | Öl | 0,86 |
| 14 | desgl. | desgl. | CH | 85° | |
| 15 | $3,4\text{-Cl}_2-C_6H_3$ | desgl. | N | 102° | |
| 16 | desgl. | desgl. | CH | Öl | 0,70 |
| 17 | $4\text{-}C_6H_5-C_6H_4$ | desgl. | N | 105° | |
| 18 | desgl. | desgl. | CH | Öl | 0,69 |
| 19 | $2\text{-Cl}-C_6H_4$ | desgl. | N | 77° | |
| 20 | desgl. | desgl. | CH | Öl | 0,67 |
| 21 | $2,4\text{-Cl}_2-C_6H_4$ | desgl. | CH | Öl | 0,67 |
| 22 | desgl. | desgl. | N | Öl | 0,83 |
| 23 | $C_6H_5$ | desgl. | N | 76–77° | |
| 24 | $C_6H_5$ | desgl. | CH | Öl | 0,64 |
| 25 | $3\text{-CN}-C_6H_4$ | desgl. | N | Öl | 0,79 |
| 26 | desgl. | desgl. | CH | Öl | 0,83 |
| 27 | $2\text{-J}-C_6H_4$ | desgl. | CH | Öl | 0,64 |
| 28 | desgl. | desgl. | N | Öl | 0,75 |
| 29 | $4\text{-Cl}-C_6H_4$ | $CH_2{=}CHCH_2(CH_3)_2C$ | N | Öl | 0,76 |
| 30 | desgl. | desgl. | CH | Öl | 0,67 |
| 31 | desgl. | $C_2H_5CH(CH_3)CH(C_2H_5)$ | N | Öl | 0,76 |
| 32 | desgl. | desgl. | CH | Öl | 0,67 |
| 33 | desgl. | $(C_6H_5)_2CH$ | CH | 127–9° | |
| 34 | desgl. | $C_6H_5CH_2(CH_3)_2C$ | CH | 133° | |
| 35 | desgl. | desgl. | N | 85° | |
| 36 | desgl. | $C_2H_5(CH_3)CH(CH_3)CH$ | N | Öl | 0,79 + 0,75 |
| 37 | desgl. | desgl. | CH | Öl | 0,69 + 0,64 |
| 38 | desgl. | $C_6H_5(CH_3)CH$ | N | Öl | 0,78 + 0,72 |
| 39 | desgl. | desgl. | CH | Öl | 0,66 + 0,62 |

Fortsetzung

| Beispiel | $R_1$ | $R_4$ | Y | Fp. | Rf-Wert |
|---|---|---|---|---|---|
| 40 | 2-Cl—$C_6H_4$ | 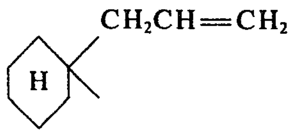 | N | Öl | 0,73 |
| 41 | $(CH_3)_3C$ | $C_6H_5CH_2(CH_3)_2C$ | CH | 80–82° | |
| 42 | desgl. | desgl. | N | 67–68° | |
| 43 | 4-Cl—$C_6H_4$ | 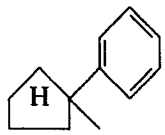 | CH | Öl | 0,71 |
| 44 | $(CH_3)_3C$ | 4-$NO_2$—$C_6H_4$— | CH | Öl | 0,68 |
| 45 | desgl. | 2,4-$Cl_2$—$C_6H_3$— | CH | Öl | 0,68 |
| 46 | desgl. | 2-Cl—$C_6H_4$ | CH | Öl | 0,65 |
| 47 | desgl. | 2,6-$Cl_2$—$C_6H_3$ | CH | 75–78° | |
| 48 | desgl. | 2-Br—$C_6H_4$ | CH | Öl | 0,65 |
| 49 | 4-Cl—$C_6H_4$ | 4-$NO_2$—$C_6H_4$ | CH | 145° | |
| 50 | 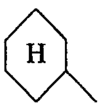 | 2,4-$Cl_2$—$C_6H_3$ | CH | Öl | 0,70 |
| 51 | 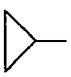 | 2,4-$Cl_2$—$C_6H_3$ | CH | 67° | |
| 52 | $(CH_3)_3CCH_2$ | desgl. | CH | Öl | 0,68 |
| 53 | $CH_2Cl(CH_3)_2C$ | 4-$NO_2$—$C_6H_4$ | CH | 56° | |
| 54 | $CH_3CH_2CH_2(CH_3)_2C$ | 2,4-$Cl_2$—$C_6H_3$ | CH | 86° | |
| 55 | $(CH_3)_2N$ | 2,4-$Cl_2$—$C_6H_3$ | CH | 81° | |
| 56 | $CH_2Cl(CH_3)_2C$ | 2,4-$Cl_2$—$C_6H_3$ | CH | 81° | |
| 57 | 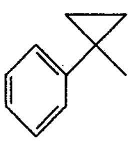 | desgl. | CH | Öl | 0,70 |
| 58 | desgl. | 4-$NO_2$—$C_6H_4$ | CH | Öl | 0,68 |
| 59 | 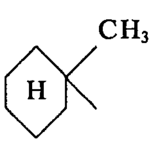 | 2,4-$Cl_2$—$C_6H_3$ | CH | Öl | 0,71 |
| 60 | desgl. | 4-$NO_2C_6H_4$ | CH | Öl | 0,71 |
| 61 | $(CH_3)_2N$ | 2,4-$Cl_2$—$C_6H_4$ | N | 103° | |

Fortsetzung

| Beispiel | $R_1$ | $R_4$ | Y | Fp. | Rf-Wert |
|---|---|---|---|---|---|
| 62 | 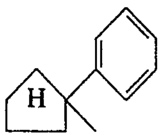 | 2,4-$Cl_2$—$C_6H_4$ | N | 78° | |
| 63 | desgl. | 2,4-$Cl_2$—$C_6H_4$ | CH | 96° | |
| 64 | desgl. | 4-$NO_2$—$C_6H_4$ | N | 92° | |
| 65 | desgl. | desgl. | CH | 122° | |
| 66 | 2-Cl—5-$NO_2$—$C_6H_3$ | $CH_3CH_2CH_2C(CH_3)_2$ | N | Öl | |
| 67 | 4-Cl—2-$CH_3$—$C_6H_3$ | desgl. | N | Öl | |
| 68 | 5-$CH_3$—2-$C_2H_5O$—$C_6H_3$ | desgl. | CH | Öl | |
| 69 | 4-$CH_3$—2-Cl—$C_6H_5$ | $C_6H_5$ | CH | Öl | |
| 70 | 2,4,5-$Cl_3$—$C_6H_2$ | $C_6H_5$ | N | Öl | |
| 71 | $C(CH_3)_3$ | $C_6H_5$—$CH_2$—$C(C_2H_5)_2$ | CH | Öl | |
| 72 | 4-Cl—$C_6H_4$— | $(n\text{-}C_3H_7)_2CH$ | CH | Öl | |
| 73 | 4-Cl—$C_6H_4$— | $C_6H_5$—$CH_2$—$C(C_2H_5)_2$ | CH | Öl | |
| 74 | desgl. | desgl. | N | Öl | |
| 75 | desgl. | n-$C_3H_7$—$C(C_2H_5)_2$ | CH | Öl | |
| 76 | desgl. | desgl. | N | 71°C | |
| 77 | desgl. | n-$C_3H_7$—$CH(C_6H_5)$ | CH | Öl | |
| 78 | desgl. | desgl. | N | 142°C | |
| 79 | desgl. | 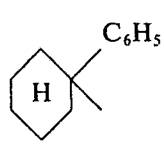 | CH | Öl | |

Fortsetzung

| Beispiel | $R_1$ | $R_4$ | Y | Fp. | Rf-Wert |
|---|---|---|---|---|---|
| 80 | 4-Cl—$C_6H_4$— | 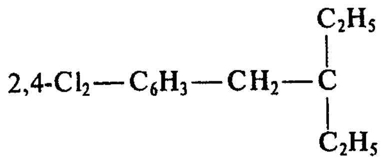 | CH | Öl | |
| 81 | desgl. | desgl. | N | Öl | |
| 82 | desgl. | 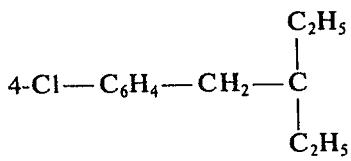 | CH | Öl | |
| 83 | desgl. | desgl. | N | Öl | |
| 84 | 2-Cl—$C_6H_5$ | $(C_6H_5)_2CH$ | CH | Öl | |
| 85 | desgl. | desgl. | N | 167°C | |
| 86 | 4-Cl—$C_6H_4$ | 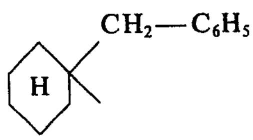 | CH | Öl | |
| 87 | desgl. | desgl. | N | Öl | |
| 88 | 2-$CH_3$—$C_6H_4$— | 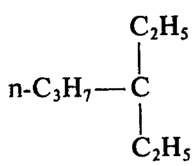 | N | Öl | |
| 89 | $C_6H_5$—NH | 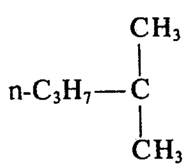 | CH | 149–150°C | |
| 90 | desgl. | desgl. | N | 126°C | |
| 91 | $(C_2H_5)_2N$ | 2,4-$Cl_2$—$C_6H_3$ | CH | 129°C | |
| 92 | $C_6H_5$—NH | 2,4-$Cl_2$—$C_6H_3$ | CH | 145°C | |

Tabelle II

Verbindungen der Formel

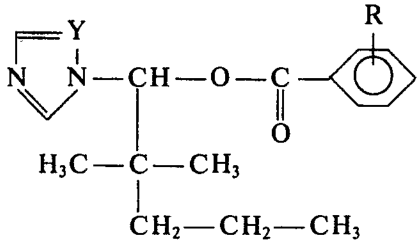

| Beispiel | R | Y | Fp. °C | Rf-Wert |
|---|---|---|---|---|
| 1 | 2-Cl, 5-$NO_2$ | CH | Öl | |
| 2 | 2-Br | CH | Öl | |
| 3 | 2-Br | N | 59 | |
| 4 | 3,4-CH=CH—CH=CH— | N | 104 | |
| 5 | 4-$C_2H_5O$ | CH | Öl | |
| 6 | 4-$C_2H_5O$ | N | Öl | |
| 7 | 2-$C_6H_5O$ | N | 74–75 | |
| 8 | 3,4-CH=CH—CH=CH— | CH | 86 | |
| 9 | 2-$NO_2$ | CH | Öl | |
| 10 | 2-NO2 | N | Öl | |
| 11 | 2-$C_6H_5O$ | CH | Öl | |
| 12 | 4-$NO_2$ | CH | Öl | |
| 13 | 3-$NO_2$ | CH | Öl | |
| 14 | 2-$CH_3O$ | N | 81 | |
| 15 | 2,4-$(CH_3O)_2$ | N | Öl | |
| 16 | 4-$N(CH_3)_2$ | N | 128 | |
| 17 | 4-$N(CH_3)_2$ | CH | 81–83 | |
| 18 | 2-$C_2H_5O$ | N | Öl | |
| 19 | 2-$C_2H_5O$ | CH | Öl | |
| 20 | 2-$CH_3$ | N | 84–85 | |
| 21 | 2-$CH_3$ | CH | Öl | |
| 22 | 2,6-$F_2$ | CH | Öl | |
| 23 | 4-$CH_3$ | CH | 95 | |
| 24 | 4-$CH_3O$ | CH | Öl | |
| 25 | 2-$CH_3O$, 4-Cl | N | Öl | |
| 26 | 3-Cl | CH | Öl | |
| 27 | 2-$CH_3O$, 4-Cl | CH | Öl | |
| 28 | 2,5-$Cl_2$ | N | 68 | |
| 29 | 2,5-$Cl_2$ | CH | Öl | |
| 30 | 3,5-$Cl_2$ | CH | Öl | |
| 31 | 2-$CH_3S$ | N | 102 | |

Fortsetzung

| Beispiel | R | Y | Fp. °C | Rf-Wert |
|---|---|---|---|---|
| 32 | 2-$CH_3S$ | CH | Öl | |
| 33 | 4-$(CH_3)_2CH-O$ | N | Öl | |
| 34 | 2-$(CH_3)_2CH-O$ | N | Öl | |
| 35 | 4-$(CH_3)_2CH-O$ | CH | Öl | |
| 36 | 2-$(CH_3)_2CH-O$ | CH | Öl | |

Tabelle III

Verbindungen der Formel I mit X gleich Schwefel

| Beispiel | $R_1$ | $R_4$ | Y | Fp. |
|---|---|---|---|---|
| 1 | $(CH_3)_2N$ | 2,4-$Cl_2-C_6H_3$ | CH | |
| 2 | $C_6H_5NH$ | 4-$NO_2-C_6H_4$ | N | |
| 3 | 4-$Cl-C_6H_4$ | n-$C_3H_7-C(CH_3)_2$ | CH | |
| 4 | desgl. | desgl. | N | |
| 5 | 2,4-$Cl_2-C_6H_3$ | desgl. | CH | |
| 6 | desgl. | desgl. | N | |
| 7 | 4-$F-C_6H_4$ | $C_6H_5-CH_2-C(CH_3)_2$ | CH | |
| 8 | 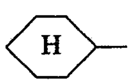 | desgl. | N | |
| 9 | $CH_2Cl-C(CH_3)_2$ | $C_2H_5-CH(CH_3)-CH(CH_3)$ | CH | |

**Patentansprüche**

1. Verbindungen der allgemeinen Formel

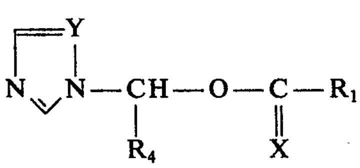 (I)

worin

$R_1$ Phenyl; Naphthyl; einen durch Halogen, Phenyl, Nitro, Cyano, $C_1-C_4$-Alkyl, $C_1-C_4$-Alkoxy, $N(C_1-C_4\text{-Alkyl})_2$, $C_1-C_4$-Alkylthio, Phenoxy, ein- bis dreifach ggf. gemischt substituierten Phenylrest; einen ggf. chlorsubstituierten $C_1-C_{10}$-Alkylrest; einen ggf. durch $C_1-C_4$-Alkyl oder Phenyl substituierten $C_3-C_7$-Cycloalkylrest; oder eine $NR_2R_3$-Gruppe;

$R_2$ Wasserstoff oder $C_1-C_4$-Alkyl;

$R_3$ $C_1-C_4$-Alkyl, Phenyl oder einen ein- bis dreifach durch Halogen, Nitro, Trifluormethyl, $C_1-C_4$-Alkyl- oder $C_1-C_4$-Alkoxy ggf. gemischt substituierten Phenylrest;

$R_4$ Phenyl; einen durch 1 bis 2 Halogenatome, die Phenyl- oder Nitrogruppe substituierten Phenylrest; einen $C_1-C_{10}$-Alkylrest; einen Alkenylrest mit bis zu 10 C-Atomen; einen durch einen oder zwei Phenylreste (die ggf. durch Halogen- oder Nitrogruppen substituiert sein können) substituierten $C_1-C_{10}$-Alkylrest; oder einen ggf. durch $C_1-C_4$-Alkyl, $C_2-C_4$-Alkenyl, Benzyl oder Phenyl substituierten $C_3-C_7$-Cycloalkylrest;

X O oder S; und

Y CH oder N

bedeutet, als Racemate, in Form der einzelnen Enantiomeren oder diastereomeren Antipodenpaare, als freie Verbindungen und als Kupferkomplexe.

2. Schädlingsbekämpfungsmittel, gekennzeichnet durch einen Gehalt an einer Verbindung nach Anspruch 1.

3. Verwendung einer Verbindung nach Anspruch 1 bei der Schädlingsbekämpfung.

4. Verwendung einer Verbindung nach Anspruch 1 bei der Bekämpfung phytopathogener Pilze.

5. Verfahren zur Herstellung von Verbindungen nach Anspruch 1, dadurch gekennzeichnet, daß man

a) ein Acyloxy- oder Thioacyloxyhalogenmethan der Formel

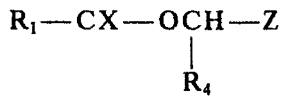

(II)

worin $R_1$, $R_4$ und X die obige Bedeutung haben und Z für Chlor oder Brom steht, mit einem Imidazol- oder Triazolsalz der Formel

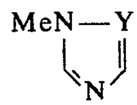

(III)

worin Me für ein Äquivalent eines Metallkations steht und Y die obige Bedeutung hat, in einem trockenen organischen Lösungsmittel umsetzt oder daß man

b) eine Verbindung der Formel

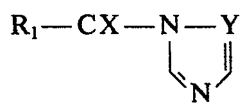

(IV)

in der $R_1$, X und Y die obige Bedeutung haben, in einem trockenen organischen Lösungsmittel mit einem Aldehyd der Formel

$R_4$—CHO (V)

worin $R_4$ die obige Bedeutung hat, umsetzt

und daß man gegebenenfalls in an sich bekannter Weise erhaltene Racemate in die Enantiomeren auftrennt oder aus entsprechenden Gemischen die diastereomeren Antipodenpaare isoliert,
und daß man gewünschtenfalls die nach den vorstehenden Verfahren erhaltenen freien Azole in an sich bekannter Weise in Kupferkomplexe überführt.

## Claims

1. Compounds of general formula

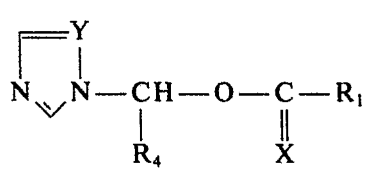

(I)

wherein

$R_1$ represents phenyl; naphthyl; a phenyl group mono- to tri-substituted and optionally substituted by a mixture of halogen, phenyl, nitro, cyano, $C_{1-4}$ alkyl, $C_{1-4}$ alkoxy, $N(C_{1-4}$ alkyl$)_2$, $C_{1-4}$ alkylthio or phenoxy; an optionally chlorine-substituted $C_{1-10}$ alkyl group; a $C_{3-7}$ cycloalkyl group optionally substituted by $C_{1-4}$ alkyl or phenyl; or $NR_2R_3$ group;

$R_2$ represents hydrogen or $C_{1-4}$ alkyl;

$R_3$ represents $C_{1-4}$ alkyl, phenyl or a phenyl group mono- to trisubstituted by halogen, nitro, trifluoromethyl, $C_{1-4}$ alkyl or $C_{1-4}$ alkoxy or by a mixture of these substituents;

$R_4$ represents phenyl; a phenyl group substituted by 1 to 2 halogen atoms or the phenyl or nitro group; a $C_{1-10}$ alkyl group; an alkenyl group with up to 10 carbon atoms; a $C_{1-10}$ alkyl group substituted by one or two phenyl groups (which may optionally be substituted by halogen or nitro groups);

or a $C_{3-7}$ cycloalkyl group optionally substituted by $C_{1-4}$ alkyl, $C_{2-4}$ alkenyl, benzyl or phenyl;
X represents O or S; and
Y represents CH or N,

as racemates, in the form of the individual enantiomers or diastereomeric pairs of enantiomers, as free compounds and as copper complexes.

2. Pesticides, characterised in that they contain a compound as claimed in claim 1.
3. Use of a compound as claimed in claim 1 for combating pests.
4. Use of a compound as claimed in claim 1 for combating phytopathogenic fungi.
5. Process for preparing compounds as claimed in claim 1, characterised in that

a) an acyloxy or thioacyloxyhalomethane of formula

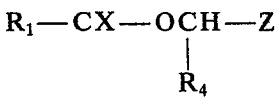
(II)

wherein $R_1$, $R_4$ and X are as hereinbefore defined and Z represents chlorine or bromine, is reacted with an imidazole or triazole salt of formula

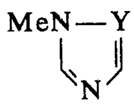
(III)

wherein Me represents an equivalent of a metal cation and Y is as hereinbefore defined, in a dry organic solvent, or

b) a compound of formula

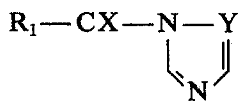
(IV)

wherein $R_1$, X and Y are as hereinbefore defined, is reacted in a dry organic solvent with an aldehyde of formula

$R_4$—CHO (V)

wherein $R_4$ is as hereinbefore defined,

and, if appropriate, any racemates obtained are resolved into their enantiomers in a manner known per se or the diastereomeric pairs of enantiomers are isolated from corresponding mixtures,
and if desired the free azoles obtained by the above-mentioned processes are converted into copper complexes in a manner known per se.

**Revendications**

1. Composés de formule générale

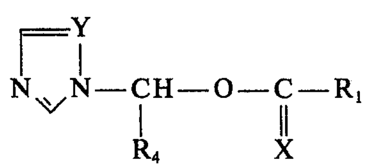
(I)

où

$R_1$ représente phényle; naphtyle; un radical phényle substitué une à trois fois éventuellement de façon mixte par halogène, phényle, nitro, cyano, alcoyle en $C_1$—$C_4$, alcoxy en $C_1$—$C_4$, N (alcoyle en $C_1$—$C_4$)$_2$, alcoylthio en $C_1$—$C_4$ en $C_1$—$C_4$, phénoxy; un radical alcoyle en $C_1$—$C_{10}$ éventuellement chlorosubstitué; un radical cycloalcoyle en $C_3$—$C_7$ éventuellement substitué par alcoyle en $C_1$—$C_4$ ou phényle; ou un groupe $NR_2R_3$;
$R_2$ représente hydrogène ou alcoyle en $C_1$—$C_4$;

$R_3$ représente alcoyle en $C_1-C_4$, phényle ou un radical phényle substitué, éventuellement de façon mixte une à trois fois par halogène, nitro, trifluorométhyle, alcoyle en $C_1-C_4$ ou alcoxy en $C_1-C_4$;

$R_4$ représente phényle; un radical phényle substitué par 1 à 2 atomes d'halogène, le groupe phényle ou nitro; un radical alcoyle en $C_1-C_{10}$; un radical alcènyle avec jusqu'à 10 atomes de C; un radical alcoyle en $C_1-C_{10}$ substitué par un ou deux radicaux phényle (qui peuvent éventuellement être substitués par des groupes halogène ou nitro); ou un radical cycloalcoyle en $C_3-C_7$ éventuellement substitué par alcoyle en $C_1-C_4$, alcènyle en $C_2-C_4$, benzyle ou phényle;

X représente O ou S; et

Y représente CH ou N,

sous forme de racémates, sous forme des énantiomères individuels ou de paires d'antipodes diastéréoisomeres, sous forme de composés libres et sous forme de complexes cupriques.

2. Agent de lutte contre les parasites, caractérisé en par une teneur en un composé selon la revendication 1.

3. Utilisation d'un composé selon la revendication 1 dans la lutte contre les parasites.

4. Utilisation d'un composé selon la revendication 1 dans la lutte contre les champignons phytopathogènes.

5. Procédé pour la préparation de composés selon la revendication 1, caractérisé en ce que

a) on fait réagir un acyloxy- ou thioacyloxyhalogénométhane de formule

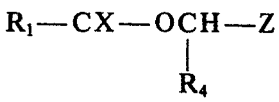 (II)

dans laquelle $R_1$, $R_4$ et X ont la signification ci-dessus et Z remplace chlore ou brome, avec un sel d'imidazole ou de triazole de formule

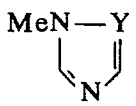 (III)

dans laquelle Me remplace un équivalent d'un cation métallique et Y a la signification ci-dessus, dans un solvant organique sec ou en ce que

b) on fait réagir un composé de formule

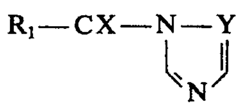 (IV)

dans laquelle $R_1$, X et Y ont la signification ci-dessus, dans un solvant organique sec, avec aldéhyde de formule

$R_4$—CHO (V)

dans laquelle $R_4$ a la signification ci-dessus

et en ce qu'éventuellement on sépare de manière en soi connue des racémates obtenus en les énantiomères ou on isole à partir de mélanges correspondants les paires d'antipodes diastéréoisomères, et en ce que si on le désire on transforme les azoles libres obtenus selon les procédés ci-dessus, de manière en soi connue, en complexes cupriques.